# EUROPEAN PATENT APPLICATION

(11) **EP 2 505 171 A1**
(43) Date of publication of application: **03.10.2012**
(21) Application number: 10833111.7
(22) Date of filing: 17.11.2010
(51) Int. Cl.: A61F 13/15, A61F 13/49, B32B 3/24

(54) **MANUFACTURING METHOD OF COMPOSITE SHEET AND MANUFACTURING DEVICE**

(30) Priority: 27.11.2009 JP 2009270634
(71) Applicant: Uni-Charm Corporation, Shikokuchuo-shi, Ehime 799-0111 (JP)
(72) Inventor: NAKAMURA, Taishi, Kanonji-shi Kagawa 769-1602 (JP); ISHIKAWA, Shinichi, Kanonji-shi Kagawa 769-1602 (JP); SHIOMI, Akihisa, Kanonji-shi Kagawa 769-1602 (JP); OKUDA, Jun, Kanonji-shi Kagawa 769-1602 (JP)
(74) Representative: Knights, Rupert
(86) International application number: PCT/JP2010/070449
(87) International publication number: WO 2011/065264

(57) **Abstract**

A method of manufacturing a stretchable composite sheet, by setting a plurality of nip areas arranged along a transport direction of a first continuous sheet, the first continuous sheet being transported while being sandwiched between rotating rolls in the nip areas, and by passing through the nip areas, as the first continuous sheet, a sheet in which stretchability is developed in the transport direction by being extended in the transport direction, including: extending the first continuous sheet in the transport direction, while transporting the first continuous sheet from a first nip area to a second nip area, the first continuous sheet being either a sheet in which stretchability has already been developed or a sheet in which stretchability has yet to be developed; forming an opening in the first continuous sheet intermittently in the transport direction, while transporting the first continuous sheet from the second nip area to a third nip area, the first continuous sheet being in a state of having been extended in the extending; and adhering in an overlapping manner a second continuous sheet having a lower stretchability than the first continuous sheet to the first continuous sheet, in the third nip area.

## Description

### Technical Field

The present invention relates to a method and an apparatus for manufacturing a stretchable composite sheet to be used in an absorbent article that absorbs fluids such as excretory fluid.

### Background Art

Until now, a two-tier stretchable composite sheet was used in an absorbent article such as a disposable diaper or a sanitary napkin (refer to PTL 1).

### Citation List

### Patent Literature

PTL 1: JP-A-2002-242064

### SUMMARY OF INVENTION

### Technical Problem

Such composite sheet is produced, for example, by joining a stretchable sheet being extended at a predetermined extension ratio to a non-stretchable sheet.
Here, there is a case in which the stretchability of the composite sheet needs to be partially weakened. For example, in a case where the composite sheet is to be used as a member that constitutes a stomach-side member or a back-side member of the diaper, from a point of view of suppressing wrinkles in an absorbent main body that constitutes a crotch member, the stretchability is required to be weakened at a portion of the composite sheet to which the absorbent main body is joined to selectively weaken an elastic force thereof.

In such a case, for example, a slit processing is selectively performed in a target portion where the stretchability is needed to be weakened in the composite sheet to form a plurality of slits piercing in a thickness direction. In this way, the stretchability is weakened in the target portion.

However, from a point of view of appearance and leakage prevention performance or the like of the composite sheet, there are cases where the slit can be formed only in the stretchable sheet and where the slit cannot be formed in the non-stretchable sheet, and in such case, above mentioned method cannot be applied.

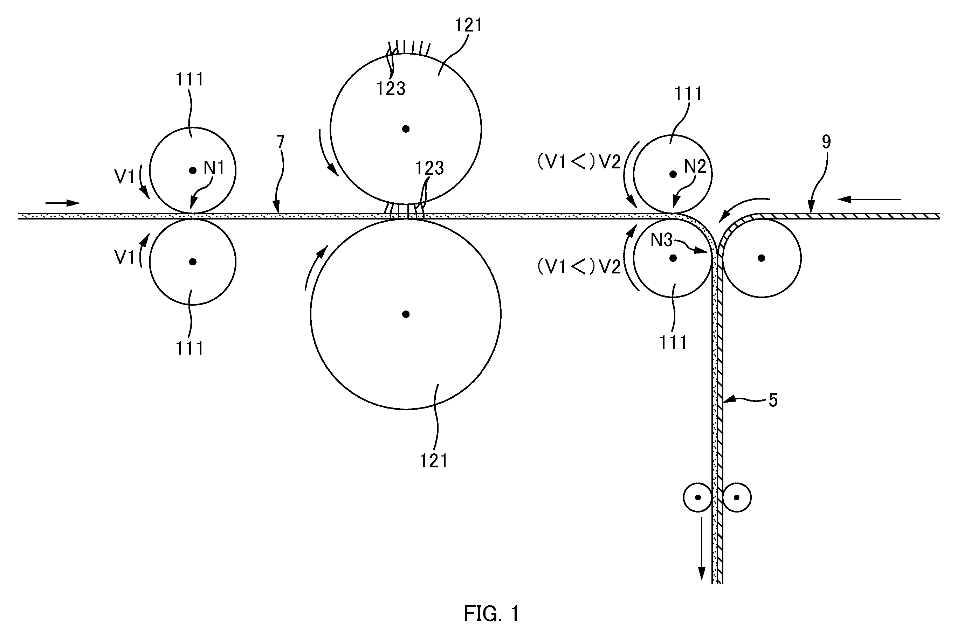

In this respect, a method shown in FIG. 1 can be considered as an example of a method that can manufacture the composite sheet without performing the slit processing on the non-stretchable sheet. First, two nip areas N1 and N2 that transport a stretchable sheet 7 while sandwiching the stretchable sheet 7 between each of the rotating rolls 111, 111 are set, and a pair of upper and lower slit rolls 121, 121 are disposed in a transport path of the sheet 7 formed between these two nip areas N1 and N2. In this method, by making a difference in peripheral speed ΔV (=V2-V1>0) between the roll 111 at the first nip area N1 and the roll 111 at the second nip area N2, the stretchable sheet 7 extends at the extension ratio while being transported from the first nip area N1 to the second nip area N2, and at the same time, the slit processing is performed by a slit blade 123 of the slit roll 121, and afterwards the stretchable sheet 7 in an extended state is adhered to a non-stretchable sheet 9 at a third nip area N3 and thereby a composite sheet 5 is produced.

However, in a case where the slit is formed in the stretchable sheet 7 while the extension of the stretchable sheet 7 is dynamically and rapidly changing (that is, while the extension ratio is increasing) in the transport path between the first nip area N1 and the second nip area N2, there is a fear that the extension ratio is imparted unstably, or the slit is formed unstably.

The present invention has been contrived in view of above conventional problems, and it is an obj ect thereof to provide a method and an apparatus for manufacturing a composite sheet that can stably impart an extension ratio or form an opening such as a slit.

### Solution to Problem

A main aspect of the invention for solving the foregoing issue is a method of manufacturing a stretchable composite sheet, by setting a plurality of nip areas arranged along a transport direction of a first continuous sheet, the first continuous sheet being transported while being sandwiched between rotating rolls in the nip areas, and by passing through the nip areas, as the first continuous sheet, a sheet in which stretchability is developed in the transport direction by being extended in the transport direction, including:
extending the first continuous sheet in the transport direction, while transporting the first continuous sheet from a first nip area to a second nip area, the first continuous sheet being either a sheet in which the stretchability has already been developed or a sheet in which the stretchability has yet to be developed;
forming an opening in the first continuous sheet intermittently in the transport direction, while transporting the first continuous sheet from the second nip area to a third nip area, the first continuous sheet being in a state of having been extended; and
adhering in an overlapping manner a second continuous sheet having a lower stretchability than the first continuous sheet to the first continuous sheet, in the third nip area.

Another aspect of the invention is
an apparatus for manufacturing a stretchable composite sheet, by setting a plurality of nip areas arranged along a transport direction of a first continuous sheet, the first continuous sheet being transported while being sandwiched between rotating rolls in the nip areas, and by passing through the nip areas, as the first continuous sheet, a sheet in which stretchability is developed in the transport direction by being extended in the transport direction, including:
rolls that form a first nip area; rolls that form a second nip area; and rolls that form a third nip area,
the first continuous sheet being extended in the transport direction while being transported from the first nip area to the second nip area, the first continuous sheet being either a sheet in which the stretchability has been developed or a sheet in which the stretchability has yet to be developed,
an opening being formed intermittently in the transport direction in the first continuous sheet, while the first continuous sheet is being transported from the second nip area to the third nip area, the first continuous sheet in a state of having been extended,
a second continuous sheet having a lower stretchability than the first continuous sheet being adhered in an overlapping manner to the first continuous sheet, in the third nip area.

Other features of the invention will become clear by the description of the present specification and the accompanying drawings.

### Advantageous Effects of Invention

According to the present invention, in manufacturing a composite sheet, it is possible to stably impart an extension ratio or form an opening.

### BRIEF DESCRIPTION OF DRAWINGS

FIG. 1 is a schematic side view of a manufacturing apparatus of a reference example.
FIG. 2A is a planar view of a composite sheet 5 that is manufactured by a method of manufacturing according to a first embodiment.
FIG. 2B is a planar view seen from the opposite side of FIG. 2A.
FIG. 2C is a cross-sectional view taken from line C-C of FIG. 2A.
FIG. 3A is a developed view of an example of a disposable diaper 10.
FIG. 3B is a cross-sectional view taken from line B-B of FIG. 3A.
FIG. 4 is a schematic side view of an apparatus 30 for manufacturing the composite sheet 5 according to the first embodiment.
FIG. 5 is an elevational view of slit rolls 41, 42.
FIG. 6 is an explanatory diagram of an orbiting belt mechanism 50 that prevents necking of a stretchable sheet 7.
FIG. 7 is an explanatory diagram of an apparatus 30a for manufacturing the composite sheet 5 according to a second embodiment.
FIG. 8 is an explanatory diagram of an apparatus 30b for manufacturing the composite sheet 5 according to a third embodiment.

### DESCRIPTION OF EMBODIMENTS

At least the following matters will become clear through the description of the present specification and the accompanying drawings.

A method of manufacturing a stretchable composite sheet, by setting a plurality of nip areas arranged along a transport direction of a first continuous sheet, the first continuous sheet being transported while being sandwiched between rotating rolls in the nip areas, and by passing through the nip areas, as the first continuous sheet, a sheet in which stretchability is developed in the transport direction by being extended in the transport direction, including:
extending the first continuous sheet in the transport direction, while transporting the first continuous sheet from a first nip area to a second nip area, the first continuous sheet being either a sheet in which the stretchability has already been developed or a sheet in which the stretchability has yet to be developed;
forming an opening in the first continuous sheet intermittently in the transport direction, while transporting the first continuous sheet from the second nip area to a third nip area, the first continuous sheet being in a state of having been extended; and
adhering in an overlapping manner a second continuous sheet having a lower stretchability than the first continuous sheet to the first continuous sheet, in the third nip area.

According to this method of manufacturing a composite sheet, the first continuous sheet is extended between the first nip area and the second nip area, and afterwards the opening is formed in the first continuous sheet that is in the extended state while being transported from the second nip area to the third nip area. Thus, since the opening is formed in a static state without any change in the extension (elongation) of the first continuous sheet, or in a quasi-static state with a small change, the formation of the opening can be performed stably. Also, the first continuous sheet is in the state of being pre-extended prior to the formation of the opening, and therefore it is hard for the extended state to be effected by the formation of the slit and the predetermined extension ratio can be stably imparted.

In the method of manufacturing a composite sheet, it is preferable that a set value of a peripheral speed of rolls that form the second nip area is faster than a set value of a peripheral speed of rolls that form the first nip area, and
a set value of a peripheral speed of rolls that form the third nip area is the same as the set value of the peripheral speed of the rolls that form the second nip area.

According to this method of manufacturing a composite sheet, the first continuous sheet is in a reliably extended state by the first nip area and the second nip area.

Also, the set value of the peripheral speed of the rolls that form the second nip area is the same as the set value of the peripheral speed of the rolls that form the third nip area. Therefore, the first continuous sheet is not further extended between the second nip area and the third nip area, and the entire length from the second nip area to the third nip area is in a static state where the extension ratio that is imparted between the above first nip area and the second nip area is maintained substantially constant. As a result, the formation of the opening can be performed stably.

In the method of manufacturing a composite sheet, it is preferable that in the forming of the opening, the first continuous sheet is wrapped around a peripheral surface of one roll of a pair of rolls, which are rotated in the transport direction as a rotating direction, at a predetermined wrapping angle and is transported by a rotation of the one roll, and while being transported, as the first continuous sheet is passed through a gap between the pair of rolls, the first continuous sheet is sandwiched and pressed between the peripheral surface of the one roll and a convex part on a peripheral surface of the other roll of the pair of rolls, and the opening is formed on the first continuous sheet, and the third nip area is set on the peripheral surface of the one roll. According to this method of manufacturing a composite sheet, the first continuous sheet is wrapped around the one roll and the third nip area is set on the peripheral surface of the one roll. That is, the first continuous sheet is wrapped around the peripheral surface of the one roll at least from a position of the gap in which the opening is formed by the convex part to the third nip area. Therefore, in a section where the first continuous sheet is likely to shrink in the width thereof (necking), that is, in a section from a position where the opening is formed in the first continuous sheet to a position where the second continuous sheet having a low stretchability is adhered, a frictional force from the peripheral surface of the one roll acts on the first continuous sheet. In this way, a shrinkage deformation of the first continuous sheet in the width direction, that is, shrinking in the width is suppressed effectively. As a result, the trouble of rupture in the first continuous sheet that might occur when forming the opening in the extended state can be prevented beforehand.

Also, in the case where the opening is formed in the state of being extended, there is a possibility that the opening might open by a tension occurring in the first continuous sheet because of such extending. However, this opening is suppressed effectively by the frictional force from the peripheral surface of the one roll.

Further, the third nip area is set on the peripheral surface of the one roll. Therefore, the one roll that should form the opening can also be used as one roll of the rolls that form the third nip area, and as a result, one of the rolls needed for forming the third nip area can be omitted and the number of apparatus components can be reduced.

In the method of manufacturing a composite sheet, it is preferable that
in the forming of the opening, the first continuous sheet is wrapped around the peripheral surface of the one roll of the pair of rolls, which are rotated in the transport direction as the rotating direction, at the predetermined wrapping angle and is transported by the rotation of the one roll, and while being transported, as the first continuous sheet is passed through the gap between the pair of rolls, the first continuous sheet is sandwiched and pressed between the peripheral surface of the one roll and the convex part on the peripheral surface of the other roll of the pair of rolls, and the opening is formed on the first continuous sheet, and
the second nip area is set on the peripheral surface of the one roll.

According to this method of manufacturing a composite sheet, the first continuous sheet is wrapped around the peripheral surface of the one roll from at least the second nip area. Thus, the shrinking in the width that may occur in the first continuous sheet from the second nip area onward can be suppressed by the frictional force of the peripheral surface acting promptly from the second nip area.

Also, the second nip area is set on the peripheral surface of the one roll. Thus, the one roll that should form the opening can also be used as one roll of the rolls that form the second nip area, and as a result, one of the rolls needed for forming the second nip area can be omitted and the number of apparatus components can be reduced.

In the method of manufacturing a composite sheet, it is preferable that
at least between a position of the gap in which the opening is formed and a position of the third nip area, both edges of the first continuous sheet in the width direction are restrained by a restraining member so as to make the first continuous sheet unable to shrink inwards in the width direction.

According to this method of manufacturing a composite sheet, the shrinking in the width of the first continuous sheet that is likely to occur because of the formation of the opening is suppressed efficiently by such restraining member restraining both edges. In this way, the trouble of rupture in the first continuous sheet because of the shrinking in the width is prevented efficiently.

Also, an apparatus for manufacturing a stretchable composite sheet, by setting a plurality of nip areas arranged along a transport direction of a first continuous sheet, the first continuous sheet being transported while being sandwiched between rotating rolls in the nip areas, and by passing through the nip areas, as the first continuous sheet, a sheet in which stretchability is developed in the transport direction by being extended in the transport direction, including:
rolls that form a first nip area; rolls that form a second nip area; and rolls that form a third nip area,
the first continuous sheet being extended in the transport direction while being transported from the first nip area to the second nip area, the first continuous sheet being either a sheet in which the stretchability is already developed or a sheet in which the stretchability is yet to be developed,
an opening being formed intermittently in the transport direction in the first continuous sheet, while the first continuous sheet is being transported from the second nip area to the third nip area, the first continuous sheet in a state of having been extended,
a second continuous sheet having a lower stretchability than the first continuous sheet being adhered in an overlapping manner to the first continuous sheet, in the third nip area.

According to such an apparatus for manufacturing a composite sheet, operational advantages that are the same as for the above-mentioned method of manufacturing can be realized.

### === First Embodiment ===

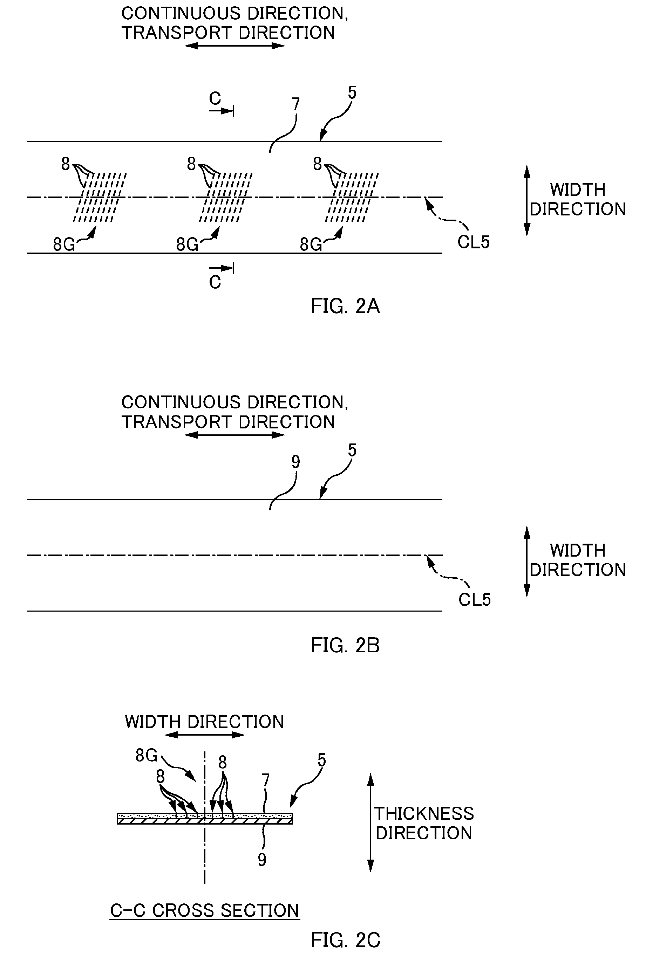

FIGS. 2A to 2C are explanatory diagrams of a composite sheet 5 manufactured by a method of manufacturing according to a first embodiment. FIG. 2A is a planar view of the composite sheet 5. FIG. 2B is a planar view seen from the opposite side of FIG. 2A. FIG. 2C is a cross-sectional view taken from line C-C of FIG. 2A.

The composite sheet 5 manufactured by the method of manufacturing according to the first embodiment is, for example, a laminate sheet 5 that is formed by stacking and adhering a stretchable sheet 7 extended at a predetermined extension ratio Re (= a length at a time of extending/a length before extending (natural length)) to a non-stretchable sheet 9 with a hot-melt adhesive or the like. And the laminate sheet 5 continues along a predetermined direction in a band-shape. And a stretchability is imparted to the composite sheet 5 in a continuous direction thereof based on the stretchability of the former stretchable sheet 7. The extension ratio Re of the stretchable sheet 7 is selected, for example, within a range of 1.5 times to 3.0 times, and in a narrower range, it is selected within a range of 2.0 times to 3.0 times.

The stretchability of the stretchable sheet 7 is developed, for example, by performing a drawing processing (extending) such as gear drawing on a whole cloth sheet of the stretchable sheet 7. Here, the "gear drawing" is a method in which the whole cloth sheet is passed through a gap between gears that are engaged with one another on peripheral surfaces of a pair of rotating upper and lower gear rolls (not shown), and then the whole cloth sheet is drawn in a rotating direction of the gear rolls that is a continuous direction of the whole cloth sheet to develop the stretchability in the whole cloth sheet. As an example of the whole cloth sheet, a nonwoven fabric or a woven fabric that includes an extensible resin fiber (fiber that can non-elastically-extend) and a stretchable resin fiber (fiber that can elastically-extend), or a resin film or the like in which an extensible resin film and a stretchable resin film are joined together can be mentioned.

On the other hand, the non-stretchable sheet 9 is a sheet that does not actually stretch. In a broad sense, the non-stretchable sheet 9 is a sheet that has lower stretchability than the stretchable sheet 7. In other words, the non-stretchable sheet 9 is a sheet that ruptures by undergoing plastic deformation with an elongation at the extension ratio Re according to the stretchable sheet 7 mentioned above, and a form of the sheet can be either of the nonwoven fabric or the woven fabric, or the film and the like.

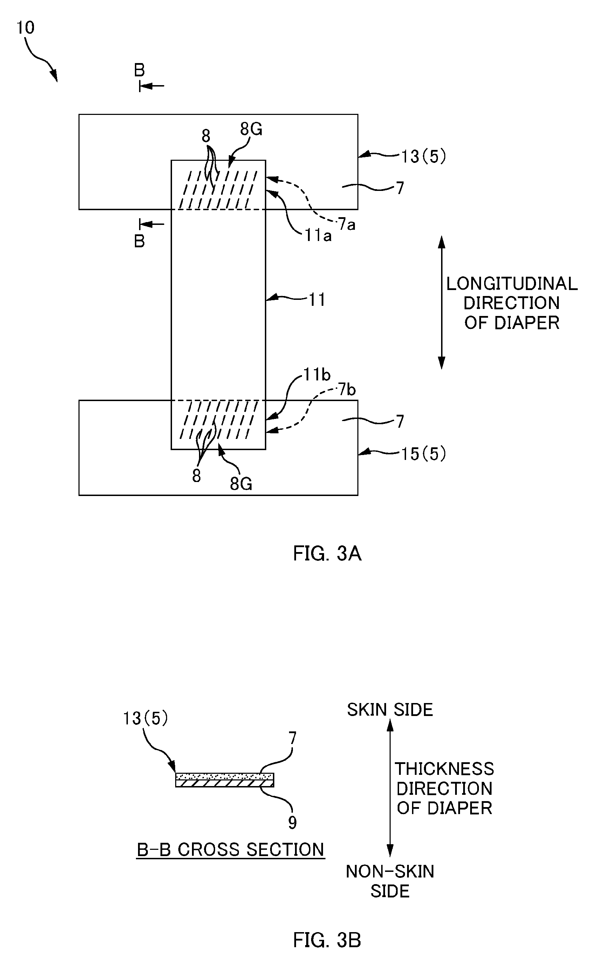

Such a composite sheet 5 is, for example, used as a part of a disposable diaper 10. FIG. 3A is a developed view of an example of the disposable diaper 10. FIG. 3B is a cross-sectional view taken from line B-B of FIG. 3A.

The diaper 10 includes an absorbent main body 11 that absorbs an excretory fluid by being applied to a wearer' s crotch, a band member on a stomach-side 13 joined to a leading-end part 11a of the absorbent main body 11 in a longitudinal direction to cover a stomach-side part of the wearer, and a band member on a back-side 15 joined to a rear-end part 11b of the absorbent main body 11 in the longitudinal direction to cover a back-side part of the wearer. The above mentioned composite sheet 5 is used as the band member on the stomach-side 13 and the band member on the back-side 15. Here, the stretchable sheet 7 of the composite sheet 5 is, for example, disposed on a side of a skin in a thickness direction of the diaper 10 and functions as a skin-side sheet. On the other hand, the non-stretchable sheet 9 is disposed on a non-skin side and functions as a leak-proof sheet. And the diaper 10 is fixed to the wearer' s body based on the stretchability of the former stretchable sheet 7.

In parts 7a, 7b that are targets for joining, in the band member on the stomach-side 13 and the band member on the back-side 15, which are joined to the absorbent main body 11, a plurality of slits 8, 8... (correspond to opening parts) are formed in a group-form. And the stretchability in the target parts 7a, 7b for joining is weakened by this group 8G of slits. This is because, in a case where an elastic force that corresponds to the stretchability in the target parts 7a, 7b for joining is large, due to this the absorbent main body 11 is wrinkled and a fluid absorption performance of the absorbent main body 11 becomes worse.

However, in a case where these slits 8, 8... are formed also on the non-stretchable sheet 9 that functions as the leak-proof sheet, it becomes a problem of the leakage prevention performance of the diaper 10. Therefore, these slits 8, 8... are formed only in the stretchable sheet 7 and are not formed in the non-stretchable sheet 9.

Therefore, as shown in FIGS. 2A and 2B, in the composite sheet 5 that is to be manufactured by the method of manufacturing according to the first embodiment, the group 8G of slits is intermittently formed in the stretchable sheet 7 in the continuous direction thereof, while the group 8G of slits is not formed in the non-stretchable sheet 9.

In the composite sheet 5, as shown in FIG. 2A, the group 8G of slits is formed in an island-form at a center part of the composite sheet 7 in a width direction. This is because the composite sheet 5 is divided into two bands at a substantially center position CL5 in the width direction, and each of the bands is respectively used as the band member on stomach-side 13 and the band member on back-side 15 in FIG. 3A.

In this regard, the slits 8, 8... are shown in solid lines in FIG. 3A, however, since the slits 8 are actually hidden in back sides (non-skin sides) of the leading-end part 11a and the rear-end part 11b of the absorbent main body 11, the slits 8 should be shown in broken lines as hidden lines. But for the sake of viewability, the slits 8 are shown in the solid lines in FIG. 3A.

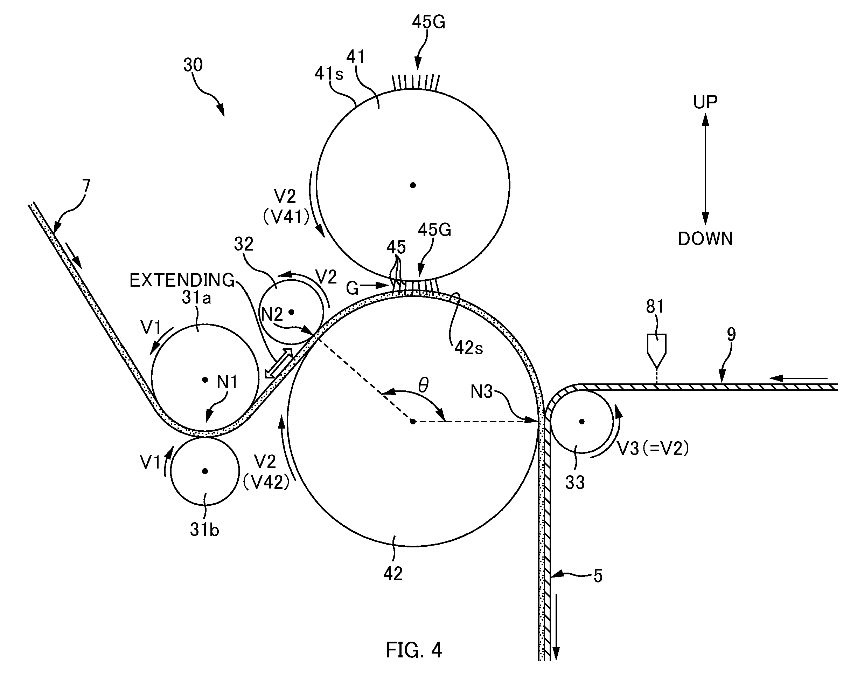

FIG. 4 is an explanatory diagram of the method of manufacturing the composite sheet 5, and is a schematic side view of an apparatus 30 for manufacturing. Hereafter, a transport direction that is the continuous direction of the stretchable sheet 7 is referred to as a MD direction, and a direction that is perpendicular to the MD direction (a direction that penetrates a paper surface in FIG. 4) is referred to as a CD direction. The CD direction is the width direction of the stretchable sheet 7, and also is the width direction of the non-stretchable sheet 9 and the composite sheet 5.

This apparatus 30 for manufacturing includes a group of a plurality of rolls 31a, 31b, 32, 33, 41, and 42 that rotate about rotational axes parallel to the CD direction. The group of rolls has three nip areas N1, N2, and N3 that are aligned along the MD direction as the transport direction in which the sheet 7 is transported while being sandwiched between the pair of rolls that are opposing one another.

In the apparatus 30 for manufacturing, the stretchable sheet 7 in which the stretchability is developed by the former gear drawing process (corresponds to a first continuous sheet) is provided in a form of a continuous sheet that continues in the transport direction. First, while the stretchable sheet 7 is being transported from the first nip area N1 to the second nip area N2, the stretchable sheet 7 is extended at the predetermined extension ratio Re in the transport direction, and while the stretchable sheet 7 is being transported from the second nip area N2 to the third nip area N3, the group 8G of slits is intermittently formed in the stretchable sheet 7 in the extended state approximately at the extension ratio Re at a predetermined pitch in the transport direction, and in the third nip area N3 the stretchable sheet 7 in the extended state is overlapped and adhered to the non-stretchable sheet 9 and thereby the composite sheet 5 is manufactured.

In this regard, a natural length of the manufactured composite sheet 5 in the transport direction, that is, an entire length of the composite sheet 5 in the natural state where no external force is acting (unloaded state) is a length in which an approximate amount of the extension ratio Re is contracted from the aforementioned extended state of the composite sheet 5. And at least in a range of the natural length to the length extended at the extension ratio Re, based on the stretchability of the stretchable sheet 7, the composite sheet 5 undergoes stretch deformation smoothly according to the strength of the external force that acts on the composite sheet 5, and this is referred to as the stretchability of the above composite sheet 5.

Hereafter, the apparatus 30 for manufacturing is explained in detail. As shown in FIG. 4, the apparatus 30 for manufacturing includes, as the aforementioned group of rolls, the rolls 31a and 31b that form the first nip area N1, the rolls 32 and 42 that form the second nip area N2, and the rolls 33 and 42 that form the third nip area N3. And between the second nip area N2 and the third nip area N3, the pair of upper and lower slit rolls 41 and 42 are disposed for performing a slit processing.

The first nip area N1 is formed by the pair of upper and lower rolls 31a and 31b. At least one roll of these rolls 31a and 31b is configured as a drive roll that drivingly rotates by a driving mechanism such as motor, however, the other roll can either be a drive roll that drivingly rotates, or a driven roll that rotates by being driven. And a nip load at the first nip area N1 (strength of a force of sandwiching between the rolls 31a and 31b) is selected, for example, within a range of 1 to 50000 Pa, and is brought about by a hydraulic cylinder or an air cylinder or the like.

On the other hand, the second nip area N2 is formed between the lower slit roll 42 and the roll 32 that is disposed opposing the lower slit roll 42, and similarly, the third nip area N3 is formed between the lower slit roll 42 and the roll 33 that is disposed opposing the lower slit roll 42. And in this way, by using the lower slit roll 42 also as a roll that forms both the second nip area N2 and the third nip area N3, the number of the rolls used can be reduced and a configuration of the apparatus can be simplified.

Here, a set value of a peripheral speed V2 of the rolls 32 and 42 that form the second nip area N2 is set higher than a set value of a peripheral speed V1 of the rolls 31a and 31b that form the first nip area N1. Specifically, the set value of the peripheral speed V2 at the second nip area N2 is set as a value of the set value of the peripheral speed V1 at the first nip area N1 multiplied by the extension ratio Re. In this way, while the stretchable sheet 7 is being transported from the first nip area N1 to the second nip area N2, the stretchable sheet 7 in a substantially natural length is extended to a length in which the substantially natural length is multiplied by the extension ratio Re. And a nip load in the second nip area N2 (strength of a force of sandwiching between the rolls 32 and 42) is selected, for example, within a range of 1 to 50000 Pa. And the roll 32 can be either of the drive roll or the driven roll.

Also, regarding the second nip area N2 and the third nip area N3, as described above, the lower slit roll 42 is commonly used as the roll that forms both the nip area N2 and the nip area N3. And according to this configuration, inevitably, a set value of a peripheral speed V3 of the rolls 33 and 42 that form the third nip area N3 becomes the same speed as the set value of the peripheral speed V2 of the rolls 32 and 42 that form the second nip area N2.

Therefore, the stretchable sheet 7 that is being transported from the second nip area N2 to the third nip area N3 is basically not further extended between the second nip area N2 and the third nip area N3, that is, the entire length from the second nip area N2 to the third nip area N3 is in a static state where the extension ratio Re that is imparted between the first nip area N1 and the second nip area N2 is maintained substantially constant. As a result, the slit processing that should be performed between these nip areas N2 and N3 can be performed stably by the slit rolls 41 and 42.

Further, before the slit processing, the stretchable sheet 7 is made into the extended state between the first nip area N1 and the second nip area N2 in advance, and therefore the extended state is hardly effected by the formation of the slits 8, and the extension ratio Re can be stably imparted.

Further, the transportation of the stretchable sheet 7 from the second nip area N2 to the third nip area N3 is performed in a state where the stretchable sheet 7 is wrapped around a peripheral surface 42s of the lower slit roll 42 at a predetermined wrapping angle θ. Thus, necking in the stretchable sheet 7 that may occur while the sheet 7 is being transported is reduced effectively by a frictional force from the peripheral surface 42s of the lower slit roll 42. And as a result, a trouble of rupture in the stretchable sheet 7 can be prevented. Further, such necking tends to be remarkable especially after the slit processing, however, the transportation of the stretchable sheet 7 after the slit processing to the third nip area N3 is also performed in the state where the stretchable sheet 7 is wrapped around the peripheral surface 42s of the lower slit roll 42. Therefore, the necking during the transportation after the slit processing is also reduced effectively.

From a point of view of reducing the necking by the frictional force, it is preferable that a friction coefficient of the peripheral surface 42s of the lower slit roll 42 is increased, that is, it is preferable that a skidproof processing is performed to the peripheral surface 42s beforehand. As an example, turning the peripheral surface 42s into a rough surface by a shot blasting process or the like, or covering the peripheral surface 42s of the lower slit roll 42 with a skidproof tape including a plurality of projections and depressions on a surface thereof is mentioned. The same skidproof processing may be performed on the rolls 31a, 31b, 32, and 33 that form the first nip area N1, the second nip area N2 and the third nip area N3.

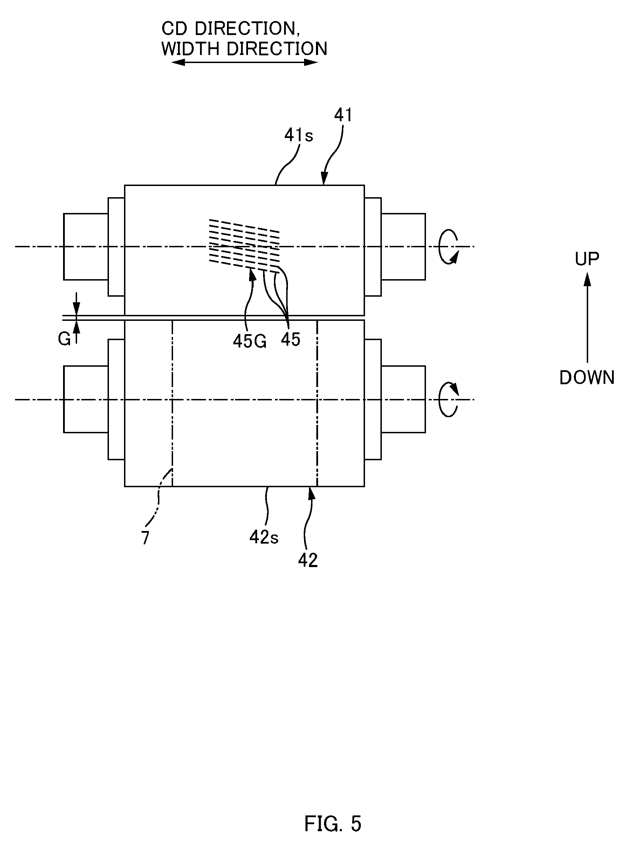

The pair of upper and lower slit rolls 41, 42 drivingly rotate by the appropriate drive mechanism such as the motor by coinciding a rotating direction thereof with the transport direction. Fig. 5 is a planar view of the slit rolls 41 and 42. The upper slit roll 41 includes a group 45G of slit blades that consists of a plurality of slit blades 45, 45... (correspond to convex parts) in a flat blade form (linear blade form), at an approximate center of the peripheral face 41s in the width direction, the group 45G of slit blades corresponding to the group 8G of slits. Also, the lower slit roll 42 is an anvil roll that receives the slit blade 45 and the peripheral surface 42s thereof is formed as a smooth surface in an area that should contact the stretchable sheet 7. Thus, as shown in FIG. 4, in a case the stretchable sheet 7 passes through a gap G between the upper slit roll 41 and the lower slit roll 42, the stretchable sheet 7 is sandwiched and pressed between the slit blades 45 and the peripheral surface 42s and thereby the slits 8 are formed by piercing the stretchable sheet 7.

Further, a radius of rotation of the slit blade 45 of the upper slit roll 41 and a radius of the peripheral surface 42s of the lower slit roll 42 can be either the same or different from each other, however, it is preferable that peripheral speeds V41 and V42 thereof are the same speed. In this way, the slit processing can be stably performed. Also, since the slit processing is performed in a state where the stretchable sheet 7 is being extended, a cutting load that is applied to the slit blade 45 during the slit processing is reduced and an operating life of the slit blade 45 is extended.

And after such slit processing, the stretchable sheet 7 passes the third nip area N3, and the non-stretchable sheet 9 (corresponds to a second continuous sheet) is also supplied to the third nip area N3 in a continuous sheet form and is overlapped and passed together with the stretchable sheet 7. Here, in a portion of the non-stretchable sheet 9 that contacts the stretchable sheet 7, the hot-melt adhesive is pre-applied by a hot-melt adhesive applying unit 81. Thus, in the case of passing the third nip area N3, the non-stretchable sheet 9 is overlapped and adhered with the stretchable sheet 7 being extended at the extension ratio Re, and thereby the composite sheet 5 is completed.

In this regard, contrary to the above, the hot-melt adhesive can be applied on the stretchable sheet 7. In such case, a setting position of the hot-melt adhesive applying unit 81 can be in an upstream side of the first nip area N1 in the transport direction, or can be between the first nip area N1 and the second nip area N2, or can be between the second nip area N2 and the third nip area N3. However, it is preferable that the roll positioned downstream than the above setting position in the transport direction and that may contact an applying surface of the stretchable sheet 7 is treated with a de-adhesion processing on a peripheral surface thereof so that the hot-melt adhesive does not transfer to the peripheral surface. As the de-adhesion processing, for example, covering the peripheral surface of the target roll with a non-adhesive tape, or performing a coating processing such as plasma processing on the peripheral surface or the like is mentioned.

Also, in either case of applying the hot-melt adhesive to the stretchable sheet 7 or to the non-stretchable sheet 9, it is preferable to apply the hot-melt adhesive by avoiding an area in which the group 8G of slits is to be formed so that the hot-melt adhesive does not adhere to such an area. In this way, the hot-melt adhesive is prevented from leaking out to a surface of the composite sheet 5 through the slits 8, and as a result a trouble of soiling a transport roll in a downstream process than the third nip area N3 due to the transferred hot-melt adhesive can be prevented.

In this regard, after passing through the third nip area N3, the stretchable sheet 7 and the non-stretchable sheet 9 are adhered together and thereby are in a state of the composite sheet 5 and a rigidity thereof is increased, and therefore hereafter the necking is suppressed by the rigidity of the composite sheet 5 itself.

Also, a nip load in the third nip area N3 (a force of sandwiching between the rolls 33 and 42) is selected, for example, within a range of 1 to 50000 Pa. And the roll 33 can be either of the drive roll or the driven roll.

Here, from a point of view of preventing the necking of the stretchable sheet 7 that is likely to occur especially after forming the slits 8, it is preferable that a restraining member is provided between a position of the gap G of the slit rolls 41 and 42 and the third nip area N3, and such restraining member restrains both edges of the stretchable sheet 7 in the width direction so that the sheet 7 is unable to shrink inwards in the width direction. In this way, the trouble of rupture in the stretchable sheet 7 because of the necking can be prevented more reliably.

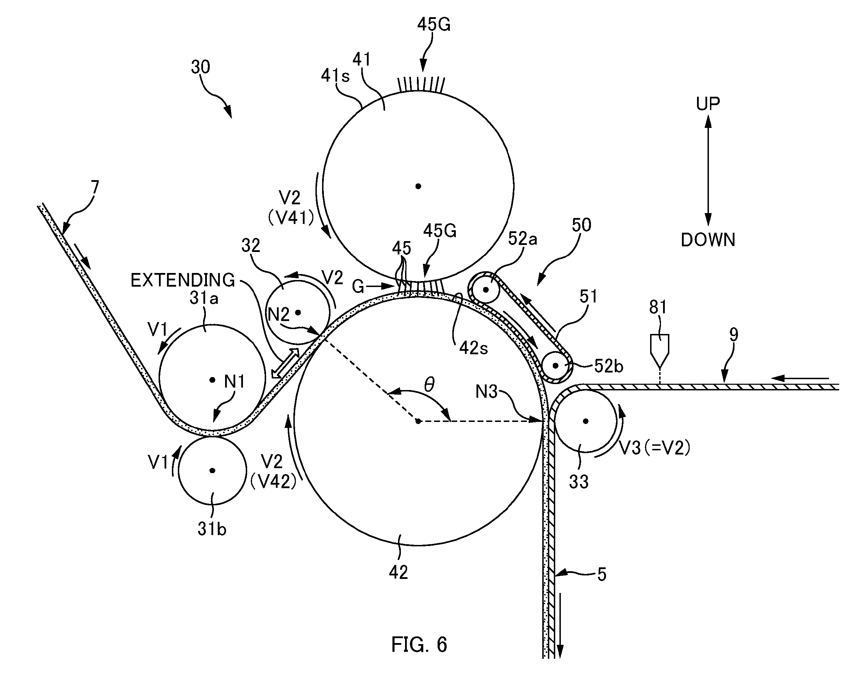

As an example of such a restraining member, an orbiting belt mechanism 50 that is provided corresponding to each edge of the lower slit roll 42 in the CD direction (width direction) is mentioned. In detail, as shown in FIG. 6, an endless belt 51 of the orbiting belt mechanism 50 is looped over suitable pulleys 52a and 52b and travels in an orbit, and these pulleys 52a and 52b are provided with a pressing force in a direction to press against the peripheral surface 42s of the lower slit roll 42. Thus, each of the endless belts 51, 51 moves in the transport direction at an approximately same speed as the peripheral speed V42 of the peripheral surface 42s without relatively sliding with respect to the peripheral surface 42s of the lower slit roll 42, while respectively pressing each of the edges of the stretchable sheet 7 to the peripheral surface 42s of the lower slit roll 42. And in this way, the stretchable sheet 7 is prevented from shrinking inward in the width direction.

Also, a similar restraining member can be disposed between the second nip area N2 and the position of the roll gap G. In this way, the trouble of rupture in the stretchable sheet 7 because of the necking can be prevented much more reliably.

### === Second Embodiment ===

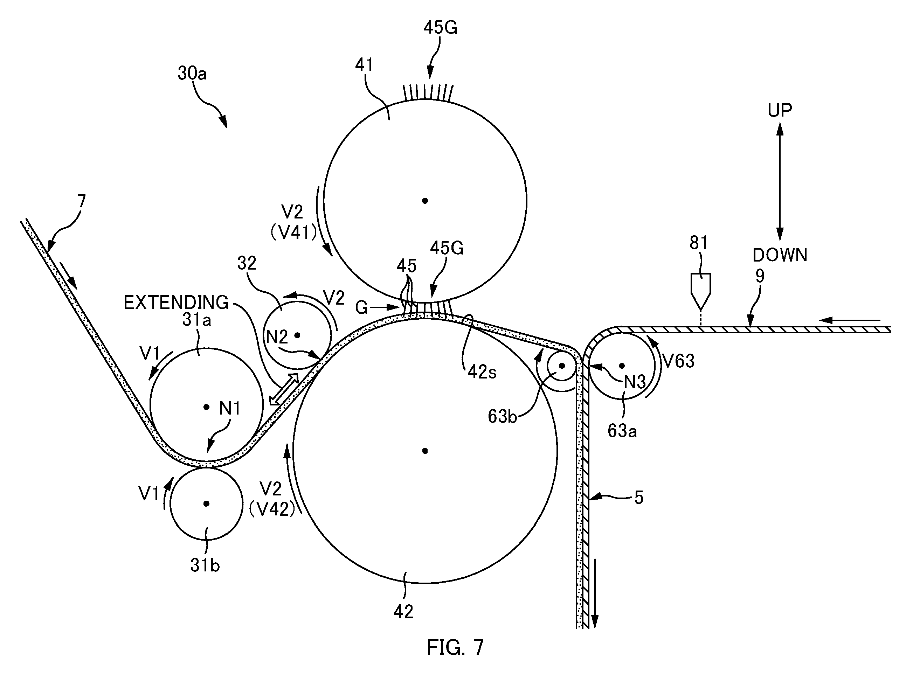

FIG. 7 is an explanatory diagram of an apparatus 30a for manufacturing according to a second embodiment. In the abovementioned first embodiment, the stretchable sheet 7 is adhered to the non-stretchable sheet 9 on the peripheral surface 42s of the lower slit roll 42 by forming the third nip area N3 on the peripheral surface 42s of the lower slit roll 42 as shown in FIG. 4. However, this second embodiment differs in that the third nip area N3 is not formed on the peripheral surface 42s of the lower slit roll 42. Further, the second embodiment is substantially the same as the aforementioned first embodiment in other respects, and therefore the same components are provided with the same symbols in FIG. 7, and explanations thereof are omitted.

As shown in FIG. 7, a pair of rolls 63a and 63b that form the third nip area N3 are disposed adjacent to the lower slit roll 42 in a position downstream of the lower slit roll 42 in the transport direction. The stretchable sheet 7 and the non-stretchable sheet 9 are adhered together by being passed through a gap between this pair of rolls 63a and 63b.

Here, at least one roll of the pair of rolls 63a and 63b is configured as the drive roll, however, the other roll can be either of the drive roll or the driven roll.

Also, in this example, a set value of a peripheral speed V63 of the driven roll of these rolls 63a and 63b is the same as the set value of the peripheral speed V42 (=V2) of the lower slit roll 42, however, depending on the circumstances, the set value of the peripheral speed V63 can be less than the set value of the peripheral speed V42 of the lower slit roll 42. That is, there is no remarkable problem if the set value is a peripheral speed in which the extension ratio Re of the stretchable sheet 7 between the second nip area N2 and the third nip areaN3 can be substantiallymaintained at its level without significantly dropping. The set value of the peripheral speed V63 can be smaller than the set value of the peripheral speed V42 (=V2) of the lower slit roll 42 in a case where, for example, the value is greater than or equal to 0.7 times the set value of the peripheral speedV42 (=V2), preferably greater than or equal to 0. 8 times, and more preferably greater than or equal to 0.9 times the set value of the peripheral speed V42.

### === Third Embodiment ===

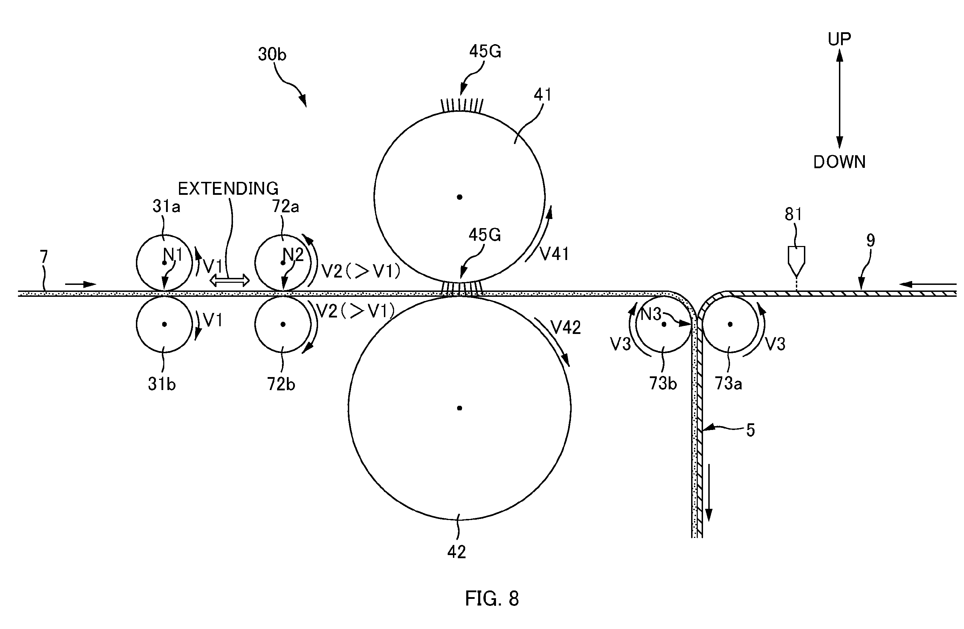

FIG. 8 is an explanatory diagram of an apparatus 30b for manufacturing according to a third embodiment. In the abovementioned first embodiment, as shown in FIG. 4, the stretchable sheet 7 is wrapped around the lower slit roll 42 at the predetermined wrapping angle θ, and the second nip area N2 and the third nip area N3 are formed on the peripheral surface 42s of the lower slit roll 42. However, the third embodiment differs in the respect that the stretchable sheet 7 is not wrapped around the lower slit roll 42, and the second nip area N2 and the third nip area N3 are not formed on the peripheral surface 42s of the lower slit roll 42. Further, the third embodiment is the same as the aforementioned first embodiment in other respects, therefore the same components are provided with the same symbols in FIG. 8, and explanations thereof are omitted.

As shown in FIG. 8, in the third embodiment the stretchable sheet 7 is not wrapped around the lower slit roll 42 and a transport path thereof is a straight path. A pair of rolls 72a and 72b that form the second nip area N2 are disposed on the upstream side of the slit rolls 41 and 42, and a pair of rolls 73a and 73b that form the third nip area N3 are disposed on the downstream side of the slit rolls 41 and 42.

Here, at least one roll of the pair of rolls 72a and 72b that form the second nip area N2 is configured as the drive roll, and the other roll is configured as either of the drive roll or the driven roll. Here, a set value of a peripheral speed V2 of the drive roll is set higher than a set value of a peripheral speed V1 of the rolls 31a and 31b that form the first nip area N1 for an amount of the extension ratio Re. In this way, while the stretchable sheet 7 is being transported from the first nip area N1 to the second nip area N2, the stretchable sheet 7 in a substantially natural length is extended to a length in which the substantially natural length is multiplied by the extension ratio Re.

In the similar way, at least one roll of the pair of rolls 73a and 73b that form the third nip area N3 is configured as the drive roll, while the other roll is configured as either of the drive roll or the driven roll.

Also, in this example, a set value of a peripheral speed V3 of the drive roll is set as the same speed as the set value of the peripheral speed V2 at the second nip area N2. Therefore, the stretchable sheet 7 that is being transported from the second nip area N2 to the third nip area N3 is basically not extended further between the second nip area N2 and the third nip area N3, that is, the entire length from the second nip area N2 to the third nip area N3 is in a static state where the extension ratio Re that is imparted between the first nip area N1 and the second nip area N2 is maintained substantially constant. As a result, the slit processing by the slit rolls 41 and 42 that should be performed between these nip areas N2 and N3 is can be performed stably.

However, depending on the circumstances, the set value of the peripheral speed V3 can be less than the set value of the peripheral speed V2. That is, there is no remarkable problem if the set value of the peripheral speed is that in which the extension ratio Re of the stretchable sheet 7 can be substantially maintained at its level without significantly dropping between the second nip area N2 and the third nip area N3. For example, the set value of the peripheral speed V3 can be smaller than the set value of the peripheral speed V2 in a case where the value is greater than or equal to 0.7 times the set value of the peripheral speed V2, preferably greater than or equal to 0.8 times, and more preferably greater than or equal to 0.9 times the set value of the peripheral speed V2. Also, in such case, set values of the peripheral speeds V41 and V42 of the upper and lower slit rolls 41 and 42 are set as for example a value between the peripheral speed V3 and the peripheral speed V2 (for example, an average value), and in this way, the stretchable sheet 7 can be stably transported at the time of the slit processing.

### === Other Embodiments ===

In the above, the embodiments according to the present invention were explained, however, the present invention is not limited to the above mentioned embodiments and modifications as described below are possible.

In the above-mentioned embodiments, the continuous sheet in which the stretchability has already been developed by the gear drawing process as a pre-process is supplied to the apparatuses for manufacturing 30, 30a, and 30b according to the present invention, however, there is no limitation to this, and the whole cloth sheet as the continuous sheet before the stretchability is developed can be supplied directly to the apparatuses for manufacturing 30, 30a, and 30b. In such case, while being transported from the first nip area N1 to the second nip area N2, in the whole cloth sheet the stretchability is developed by being extended at the extension ratio Re, and thereby the whole cloth sheet becomes the stretchable sheet 7.

In the above-mentioned embodiments, the slit 8 is mentioned as an example of an opening that is formed on the stretchable sheet 7, and the slit 8 is formed by the slit blade 45 in a flat blade form. However, there is no limitation to this. For example, a shape of the opening does not have to be linear, and a shape having a circle or polygon area and the like is possible. In such case, instead of the upper slit roll 41, an emboss roll including an embossed convex part having a shape that corresponds to the opening is used.

In the above-mentioned embodiments, the case is mentioned as an example in which the group 8G of slits is selectively formed only in the target parts 7a, 7b for joining in the stretchable sheet 7. That is, the group 8G of slits is intermittently formed in the stretchable sheet 7 in the transport direction as the continuous direction thereof. However, there is no limitation to this, and for example, the plurality of slits 8, 8... can be formed by distributing uniformly through an entire length of the stretchable sheet 7 in the transport direction.

In the above-mentioned embodiments, a control section for controlling rotations of rolls to be the drive roll of the rolls that form each of the nip areas N1, N2 and N3 or the slit rolls 41 and 42 is not explained. However, as for an example of the control section an appropriate sequencer or a computer or the like can be mentioned. It goes without saying that the control section performs an appropriate speed control such as feedback control so that the actual peripheral speed of each of the rolls becomes the set value.

In the above-mentioned embodiments, the nonwoven fabric or the woven fabric or the like that includes the stretchable resin fiber and the extensible resin fiber is mentioned as an example of a material of the whole cloth sheet of the stretchable sheet 7. It is mentioned that the extensible resin fiber is "a fiber that can non-elastically-extend", in other words, the extensible resin fiber can be mentioned as "a fiber that undergoes plastic deformation by an elongation smaller than an elongation of the stretchable resin fiber at an elastic limit". An example of the extensible resin fiber is a thermoplastic polyolefin fiber, and an example of the stretchable resin fiber is a thermoplastic elastomer fiber. Examples of the thermoplastic polyolefin fiber include single fibers, such as a polypropylene fiber and a polyester fiber, and a conjugate fiber with a sheath core structure consists of polypropylene or polyester, and examples of the thermoplastic elastomer fiber include a polyurethane fiber for example.

### Reference Signs List

5 laminate sheet (composite sheet), 7 stretchable sheet (first continuous sheet), 7a target part for joining, 7b target part for joining, 8 slit (opening), 8G group of slits, 9 non-stretchable sheet (second continuous sheet), 10 disposable diaper, 11 absorbent main body, 11a leading-end part, 11b rear-end part, 13 band member on stomach-side, 15 band member on back-side, 30 apparatus for manufacturing, 30a apparatus for manufacturing, 30b apparatus for manufacturing, 31a roll, 31b roll, 32 roll, 33 roll, 41 upper slit roll, 41s peripheral face, 42 lower slit roll, 42s peripheral face, 45 slit blade (convex part), 45G group of slit blades, 50 orbiting belt mechanism, 51 endless belt, 52a pulley, 52b pulley, 63a roll, 63b roll, 72a roll, 72b roll, 73a roll, 73b roll, 81 hot-melt adhesive applying unit, N1 first nip area, N2 second nip area, N3 third nip area, G roll gap

## Claims

1. A method of manufacturing a stretchable composite sheet, by setting a plurality of nip areas arranged along a transport direction of a first continuous sheet, the first continuous sheet being transported while being sandwiched between rotating rolls in the nip areas, and by passing through the nip areas, as the first continuous sheet, a sheet in which stretchability is developed in the transport direction by being extended in the transport direction, comprising:
extending the first continuous sheet in the transport direction, while transporting the first continuous sheet from a first nip area to a second nip area, the first continuous sheet being either a sheet in which stretchability has already been developed or a sheet in which stretchability has yet to be developed;
forming an opening in the first continuous sheet intermittently in the transport direction, while transporting the first continuous sheet from the second nip area to a third nip area, the first continuous sheet being in a state of having been extended; and
adhering in an overlapping manner a second continuous sheet having a lower stretchability than the first continuous sheet to the first continuous sheet, in the third nip area.

2. A method of manufacturing a composite sheet according to claim 1, wherein
a set value of a peripheral speed of rolls that form the second nip area is faster than a set value of a peripheral speed of rolls that form the first nip area, and
a set value of a peripheral speed of rolls that form the third nip area is the same as the set value of the peripheral speed of the rolls that form the second nip area.

3. A method of manufacturing a composite sheet according to claim 2, wherein
in the forming of the opening, the first continuous sheet is wrapped around a peripheral surface of one roll of a pair of rolls, which are rotated in the transport direction as a rotating direction, at a predetermined wrapping angle and is transported by a rotation of the one roll, and while being transported, as the first continuous sheet is passed through a gap between the pair of rolls, the first continuous sheet is sandwiched and pressed between the peripheral surface of the one roll and a convex part on a peripheral surface of the other roll of the pair of rolls, and the opening is formed on the first continuous sheet, and
the third nip area is set on the peripheral surface of the one roll.

4. A method of manufacturing a composite sheet according to claim 2 or 3, wherein
in the forming of the opening, the first continuous sheet is wrapped around the peripheral surface of the one roll of the pair of rolls, which are rotated in the transport direction as the rotating direction, at the predetermined wrapping angle and is transported by the rotation of the one roll, and while being transported, as the first continuous sheet is passed through the gap between the pair of rolls, the first continuous sheet is sandwiched and pressed between the peripheral surface of the one roll and the convex part on the peripheral surface of the other roll of the pair of rolls, and the opening is formed on the first continuous sheet, and
the second nip area is set on the peripheral surface of the one roll.

5. A method of manufacturing a composite sheet according to any of claims 1 to 4, wherein
at least between a position of the gap in which the opening is formed and a position of the third nip area, both edges of the first continuous sheet in the width direction are restrained by a restraining member so as to make the first continuous sheet unable to shrink inwards in the width direction.

6. An apparatus for manufacturing a stretchable composite sheet, by setting a plurality of nip areas arranged along a transport direction of a first continuous sheet, the first continuous sheet being transported while being sandwiched between rotating rolls in the nip areas, and by passing through the nip areas, as the first continuous sheet, a sheet in which stretchability is developed in the transport direction by being extended in the transport direction, comprising:
rolls that form a first nip area; rolls that form a second nip area; and rolls that form a third nip area,
the first continuous sheet being extended in the transport direction while being transported from the first nip area to the second nip area, the first continuous sheet being either a sheet in which stretchability has already been developed or a sheet in which stretchability has yet to be developed,
an opening being formed intermittently in the transport direction in the first continuous sheet, while the first continuous sheet is being transported from the second nip area to the third nip area, the first continuous sheet in a state of having been extended,
a second continuous sheet having a lower stretchability than the first continuous sheet being adhered in an overlapping manner to the first continuous sheet, in the third nip area.
